# EUROPEAN PATENT APPLICATION

(11) **EP 1 731 178 A2**
(43) Date of publication of application: **13.12.2006**
(21) Application number: 06113495.3
(22) Date of filing: 04.05.2006
(51) Int. Cl.: A61L 27/54, A61L 27/58, A61L 31/14, A61L 31/16

(54) **Resorbable polymer composition, implant and method of making implant**

(30) Priority: 10.05.2005 US 125814
(71) Applicant: UNIVERSITY OF ZURICH, 8600 Zürich (CH)
(72) Inventor: Weber, Franz, 78224, DE-Singen (DE)
(74) Representative: Lax, Monica Ingeborg

(57) **Abstract**

Novel polymer compositions that are useful in the manufacture of medical implants, implants having osteogenic properties and methods of making the implants are disclosed. Polymer compositions include a base material having a polymer matrix of resorbable polymer(s) or copolymer(s), and a glycerol mono-, di-, or triester derivative, wherein the glycerol mono-, di-, or triester derivative is present in an amount imparting osteogenic properties for the composition.

## Description

### FIELD OF THE INVENTION

The present invention relates to novel polymer compositions that are useful in the manufacture of medical implants. More particularly, embodiments of the invention relate to polymer compositions having osteogenic properties. The polymer compositions are biodegradable or bioresorbable and they can be fashioned into medical implants for implantation in the body. Implants having osteogenic properties and methods of making said implants are also disclosed.

### BACKGROUND OF THE INVENTION

The healing process of bone is a complicated cascade of events. Rapid and diverse events are activated by a fracture or osteotomy of a bone in order to limit the loss of blood and initiate cellular migration resulting in repair. Current concepts suggest that these cellular events are controlled to a large part by growth factors, low-molecular-weight glycoproteins, inducing migration, proliferation and differentiation of an appropriate subset of cells in the site of the fracture.

Despite of the amount of known details the bone healing as a whole is still a poorly understood process. Based on this lacking information and experimental data research has revealed several methods to enhance bone growth, such as mechanical stimulation, electromagnetic fields, low-intensity ultrasound, osteoconductive materials, for instance hydroxyapatite, tri-calcium phosphate (TCP), bioactive glass etc., and osteoinductive materials, such as growth factors.

Osteoinduction is a process where any substance, stimulation etc. starts or enhances a cellular response resulting in a bone formation process. Growth factors are a wide group of molecules known to possess this effect. According to the current knowledge, bone morphogenetic proteins (BMP) are the only growth factors known to induce bone formation heterotopically by inducing undifferentiated mesenchymal cells to differentiate into osteoblasts. Consequently, several BMPs are shown to boost the bone healing process when supplementary doses are given.

For example, US Patent No. 5,725,491 discloses a biodegradable film dressing as a delivery system of various therapeutic agents, such as BMPs. The therapeutic agent is delivered from the film dressing in a certain and controlled release rate. However, BMPs are produced by genetic engineering, which is still rather expensive. Also, delivery of a correct dose of BMPs is difficult and presents great challenges for the future.

Known materials, methods and implants are expensive and exploitation of such materials, methods and implants is constrained.

US patent publication 2003-0104029 A1 discloses that the rigidity of biodegradable polymers can be softened and their flexibility increased by a treatment with a known plasticizer, N-methyl-2-pyrrolidone (NMP), to an extent that allows their use in GBR. Additionally, it is disclosed that NMP itself has an unexpected bone formation inducing effect.

US Patent No. 5,888,533 discloses a flowable composition for forming a solid biodegradable implant *in situ* within the body. This composition contains a non-polymeric water-insoluble biodegradable material and a biocompatible solvent, for instance NMP, and may additionally contain a release rate-modifying agent, such as glycerol triacetate (triacetin). A non-polymeric system, which is suitable as a controlled release implant, is also disclosed. There is no mention or suggestion of a possible bone formation inducing activity of triacetin.

US patent Application No. 6,162,258 discloses a method for treating monolithic bone, wherein for instance polyhydroxy esters, such as monoacetin and triacetin, are used as a mechanical strength-conserving agent. There is no mention or suggestion of a possible bone formation inducing activity of these esters.

### BRIEF DESCRIPTION OF THE INVENTION

An object of the present invention is to provide novel resorbable polymer compositions having osteogenic properties so as to alleviate the above disadvantages. Another object is to provide novel resorbable implants having osteogenic properties. A further object is to provide methods of making resorbable implants having osteogenic properties.

These objects are achieved by providing resorbable polymer compositions, resorbable implants and methods of making resorbable implants comprising a base material including a polymer matrix of resorbable polymer(s) or copolymer(s) and a glycerol mono-, di-, or triester with a carboxylic acid having 1 to 6 carbon atoms, in particular a glycerol di-, or triester with a carboxylic acid having 1 to 4 carbon atoms, such as glyceryl triacetate, i.e., triacetin. In the present context, the common terms "triacetin" and "diacetin" are alternatively used of glyceryl triacetate and glyceryl diacetate, respectively.

According to one embodiment of the invention, the polymer matrix comprises Polylactide/Polyglycolide/Trimethylene carbonate copolymer (PLA/PGA/TMC) with a composition of 80/10/10.

According to another embodiment of the invention, the polymer matrix comprises Poly D, L-lactide/Poly L-lactide/Trimethylene carbonate copolymer (PLDLA/PLA/TMC) with a composition of 55/40/5.

According to a third embodiment of the invention, the polymer matrix comprises 80 wt-% P(L/DL)LA (70/30) and 20 wt-% PLLA/TMC (70/30).

According to a fourth embodiment of the invention, the implant is a membrane.

According to one embodiment of the method of the invention, the method comprises the steps of selecting polymer(s) or copolymer(s) of a polymer matrix of the implant, mixing said polymer(s) or copolymer(s) to form the polymer matrix, forming the implant from said polymer matrix, and adding a glycerol mono-, di-, or triester with a carboxylic acid having 1 to 6 carbon, to the implant in an amount imparting osteogenic properties for said implant. In a particularly preferred embodiment of the method of the invention, glycerol di-, and triesters with carboxylic acids having 1 to 4 carbon atoms, more preferably 2 to 3 carbon atoms, namely acetic acid and propionic acid, such as glyceryl triacetate and glyceryl diacetate, and most preferably glyceryl triacetate, i.e., triacetin, are used.

According to another embodiment of the method of the invention, the method comprises the steps of selecting polymer(s) or copolymer(s) of a polymer matrix of the implant, adding a glycerol mono-, di-, or triester with a carboxylic acid having 1 to 6 carbon atoms, to the polymer matrix in an amount imparting osteogenic properties for the implant, and forming the implant from the mixture of said polymer matrix and said glycerol mono-, di-, or triester. In a particularly preferred embodiment of the method of the invention, glycerol di-, and triesters with carboxylic acids having 1 to 4 carbon atoms, more preferably 2 to 3 carbon atoms, such as glyceryl triacetate and glyceryl diacetate, and most preferably glyceryl triacetate, i.e., triacetin, are used.

According to one embodiment of the method of the invention the glycerol mono-, di-, or triester derivatives are chemically coupled in a conventional manner known to a person skilled in the art to the implant and released over time as mono-, di- or triacylglycerols.

An advantage of polymer compositions, implants and methods of the invention is that substantially inexpensive products are achieved as compared with known solutions enhancing bone healing.

### BRIEF DESCRIPTION OF THE DRAWINGS

In the following the invention will be described in greater detail by means of preferred embodiments with reference to the attached drawings, in which:
Figure 1a shows radiographs of rat calvarial bones where critical size defects were left untreated, Figure 1b shows rat calvarial bones, where said defect is covered by a (PLA/PGA/TMC) 80/10/10 membrane, and Figure 1c shows rat calvarial bones, where said defect is covered by a (PLA/PGA/TMC) 80/10/10 membrane and treated with triacetin as described in Example 1; and
Figure 2 shows the effect of different solvents in comparison with triacetin on the alkaline phosphate activity of preosteoblastic cells.

The present invention relates to a combination of a glycerol mono-, di-, or triester with a carboxylic acid having 1 to 6 carbon atoms, preferably glycerol di-, and triesters with carboxylic acids having 1 to 4 carbon atoms, more preferably 2 to 3 carbon atoms, such as glyceryl triacetate and glyceryl diacetate, and most preferably glyceryl triacetate, i.e., triacetin, and resorbable polymers or copolymers. The invention is based on the unexpected realization that by combining a resorbable matrix material and known plasticizer, glyceryl triacetate or triacetin, in a certain ratio, an implant having osteogenic properties is achieved. The implant thus induces bone growth due to the osteogenic properties of the polymer composition and enhances bone healing after osteotomies and bone fractures.

The implant forms include, but are not limited to, membranes, films, plates, mesh plates, screws, taps or other formed pieces.

The implant can be prepared for example of polyglycolide, polylactides, polycaprolactones, polytrimethylenecarbonates, polyhydroxybutyrates, polyhydroxyvalerates, polydioxanones, polyorthoesters, polycarbonates, polytyrosinecarbonates, polyorthocarbonates, polyalkylene oxalates, polyalkylene succinates, poly(malic acid), poly(maleic anhydride), polypeptides, polydepsipeptides, polyvinylalcohol, polyethylene glycols, polyesteramides, polyamides, polyanhydrides, polyurethanes, polyphosphazenes, polycyanoacrylates, polyfumarates, poly(amino acids), modified polysaccharides (like cellulose, starch, dextran, chitin, chitosan, etc.), modified proteins (like collagen, casein, fibrin, etc.) and their copolymers, terpolymers or combinations or mixtures or polymer blends thereof. Polyglycolide, poly(L-lactide-co-glycolide), poly(D,L-lactide-co-glycolide), poly(L-lactide), poly(D,L-lactide), poly(L-lactide-co-D,L-lactide), polycaprolactone, poly(L-lactide-co-caprolactone), poly(D,L-lactide-co-caprolactone) polytrimethylenecarbonate, poly(L-lactide-co-trimethylenecarbonate), poly(D,L-lactide-co-trimethylenecarbonate), polydioxanone and copolymers, terpolymers and polymer blends thereof are highly preferred polymers.

### EXAMPLE 1

Polylactide/Polyglycolide/Trimethylene carbonate copolymer (PLA/PGA/TMC), with a composition of 80/10/10, granulates were compression moulded to form a film with a thickness of 0.2 mm. Used compression temperature was 180°C and pressure 130 bar. From the film 5 rectangular pieces were cut.

The weight of the individual film pieces was measured with balance with an accuracy of 1 mg. The film pieces were then immersed individually into triacetin for 24h. After immersion the surplus of triacetin was removed, the film pieces were air dried for 20 minutes and the weight of the pieces was measured again.

The weight of the film pieces before and after immersion into triacetin are shown in Table 1. The average amount of triacetin diffused into polymeric film was 50.1 %.

**Table 1. Weight of the film pieces before and after immersion into triacetin**

| Weight before immersion into triacetin (mg) | Weight after immersion into triacetin (mg) | Triacetin content (mg) | Polymer content (%) | Triacetin content (%) |
|---|---|---|---|---|
| 70.6 | 139.7 | 69.1 | 50.5 | **49.5** |
| 82.4 | 165.9 | 83.5 | 49.7 | **50.3** |
| 74.2 | 151.1 | 76.9 | 49.1 | **50.9** |
| 70.8 | 140.8 | 70.0 | 50.3 | **49.7** |

GTR membranes produced with the method described here in were used in a comparable rat study disclosed in Example 2.

### EXAMPLE 2

This rat study shows the osteogenetic effect of PLA/PGA/TMC and PLDLA/PLA/TMC membranes when treated with triacetin. The details of the tested membranes can be found in the following table 2.

**Table 2. Materials and codes of Example 2**

| Code | Materials |
|---|---|
| E1M-11® triacetin | PLA/PGA/TMC (80/10/10), treated with triacetin (see Example 1) |
| E1M-11® | PLA/PGA/TMC (80/10/10) |

The study design included 4 rats with 8-mm artificial craniotomy defects each. The defects were treated with biodegradable membranes. The matrixes of the resorbable membranes are presented in Table 2.

The rats were sacrificed 5 weeks after the operation and the calvarial bone excised. Bone regeneration was determined by radiography.

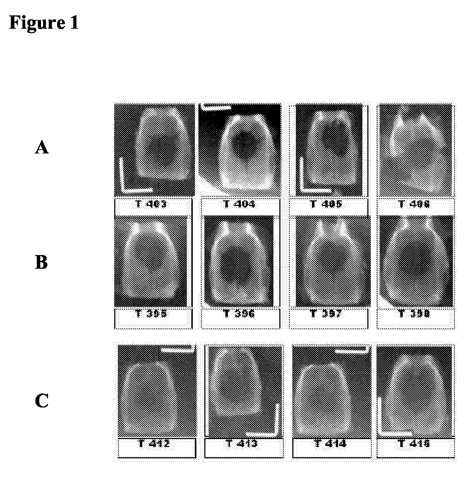

Figures 1a to 1c illustrate the results showing that triacetin treated membranes enhance bone regeneration determined by radiography.

### EXAMPLE 3

According to one embodiment of the method of the present invention, a glycerol mono-, di-, or triester with a carboxylic acid having 1 to 6 carbon atoms, such as triacetin, is added to the polymer matrix that has been already fashioned into the form of a medical implant.

Polymer compositions were prepared by dry-mixing commercially available granular-form base materials with commercially available copolymer additives. The material composition was 80 wt-% P(L/DL)LA (70/30) and 20 wt-% PLLA/TMC (70/30). The components were weighed according to a desired weight ratio into a container, which was then rotated in a Turbula T2F shaker mixer for 30 minutes until a homogenous dry mixture was obtained. The resulting mixture was then dried in vacuum at 60°C for 8 to 12 hours and thereafter melt-blended and injection-moulded in to plate-shaped test pieces. The injection-moulding machine used was a fully electric Fanuc Roboshot Alpha i30A injection-moulding machine with a mould clamping force of 300 kN. The injection unit was equipped with high speed (max. 66 cm³/s to 330 mm/s), high pressure (max. 2500 bar) injection options. The barrel diameter was 16 mm and it was equipped with three-band heater zones, a standard profile anticorrosion screw and a standard open nozzle with a 2.5 mm hole. The extruder melt-blending and homogenization conditions of the material during the metering phase of the process included a back pressure of 40 to 60 bar, a screw speed of 60 to 100 rpm and barrel temperatures of 160 to 230°C. injection moulding conditions included a nozzle temperature of 180 to 230°C, an injection speed of 80 to 300 mm/s, a maximum injection pressure of 2500 bar, a pack pressure of 1000 to 2300 bar for 3 to 8 s, a cooling time of 10 to 22 s and a mould temperature of 20 to 30°C. The total cycle time was 20 to 40 s consisting of the following phases during one injection-moulding process cycle: closing of the mould, injection of the molten polymer into the mould, pack pressure, cooling while extruder was metering for the next cycle during cooling phase, opening the mould and ejection of article from the mould.

The plates were sterilized by gamma irradiation with a nominal dose of 25 kGy. After sterilisation, the entire plates or rectangular fractions were submerged in triacetin (1,2,3-Triacetoxypropane, 1,2,3-Triacetylglycerol, >98,5%, Sigma Inc., USA) for 24 h. Resorbable polymer matrix absorbs triacetin when immersed into it. Following the incubation, the membrane is placed in a holder so that the surplus of triacetin is drains off. Thereafter, an implant loaded with triacetin is implanted into the body, and triacetin is released gradually during a certain period of time. If the rate of releasing is appropriate, triacetin owns osteogenic properties. As with almost any pharmaceuticals, the concentration of triacetin must be within certain limits, called a therapeutic window. Below the window, triacetin is inefficacious. Correspondingly, above the window, triacetin presents an adverse event by inhibiting certain proteins, other molecules or cell lines. The triacetin content is preferably between 0.05 and 60 weight-%, more preferably between 10 and 50 weight-%.

According to one preferred embodiment of the method of the present invention, a glycerol mono-, di-, or triester with a carboxylic acid having 1 to 6 carbon atoms, such as triacetin, is mixed with a polymer matrix or one of its components before the polymer matrix is fashioned into the form of a medical implant. The mixing can take place in an extruder, in a mixer or similar equipment known per se.

Triacetin may be applied to the implant as well by packing said implant into a container with triacetin already in the production process. Triacetin will be absorbed to the polymer matrix of the implant during storage in said container.

The polymer composition of the present invention can be fashioned into implants by injection moulding, compression moulding, extrusion or with another melt-moulding process known by persons skilled in the art.

### EXAMPLE 4

Example 4 presents one preferred embodiment of the present invention, where the implant is a barrier membrane in Guided Tissue Regeneration (GTR) to treat a periodontal defect.

The membrane comprises PLA/PGA-matrix polymers. The membrane is packaged in a slot of a package, such as a plastic blister. The preparation of the membrane is conducted during manufacturing to generate a membrane pre-soaked with triacetin or as one stage of surgical operation as follows:
1. After opening the package, a proper amount of triacetin is poured into the membrane slot. The membrane is fully immersed in triacetin for an adequate period, for example 5 minutes to 60 minutes, preferably for 60 minutes.
2. The membrane is removed from the slot.
3. The surplus of triacetin is wiped away.
4. The membrane is ready for use as a barrier between the gingival soft tissue and the healing bone tissue and/or periodontal tissues in order to prevent the gingival soft tissue filling the defect side. In the conditions of a normal operating theater temperature and humidity, the membrane stays malleable for several hours.

### EXAMPLE 5

To compare the effect of various plasticizer regarding their osteogenetic effect to that of triacetin, MC3T3-E1 cells were grown in an alpha-modified Minimum Essential Medium (Life Technologies, Inc., Grand Island, NY) containing 10% fetal calf serum (Life Technologies, Inc.), 50µg/ml gentamycin, and 50µg/ml ascorbic acid. To examine the action of triacetin on this cell line, 1x10⁵ cells per well were plated in 6-well plates and triacetin subsequently added. Medium exchange was performed after 3 days and alkaline phosphatase (ALP) was determined according to Lowry, O. et al. [J. Biol. Chem. 207 (1954) 19-37]: p-nitrophenol liberated was converted to p-nitrophenylate by adding 400 µl of 1 M NaOH, which was quantitated by measuring the absorbance at 410 nm (epsilon=17500/molxcm). Alkaline phosphatase activity was normalized to total protein and expressed as nmol nitrophenylate generated per min per mg protein.

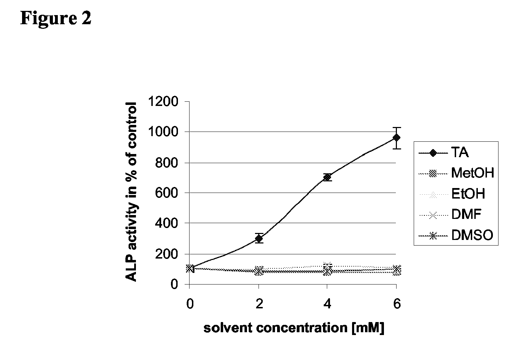

MC3T3-E1 cells were treated with increasing amounts of different solvents and the effect on the maturation of MC3T3-E1 cells determined after 6 days by alkaline phosphatase activity. The results are shown in Figure 2.

The maturation of MC3T3-E1 cells increases in a concentration dependent manner with the amount of triacetin applied ranging from 0 to 6 mM of triacetin. Other solvents like methanol (MeOH), ethanol (EtOH), N, N-dimethylformamide (DMF) and dimethylsulfoxide (DMSO) showed no positive effect on the maturation of preosteoblastic cells.

Implants of the invention can be used for example in guided bone regeneration applications, where the effect of a triacetin loaded barrier membrane is required to avoid soft tissue in-growth or muscle prolapse in the area where new bone formation is required, and to enhance bone regeneration.

It will be obvious to a person skilled in the art that, as the technology advances, the inventive concept can be implemented in various ways. The invention and its embodiments are not limited to the examples described above but may vary within the scope of the claims.

## Claims

1. A resorbable polymer composition comprising:
a base material including a polymer matrix of resorbable polymer or copolymer, and
a glycerol mono-, di-, or triester with a carboxylic acid having 1 to 6 carbon atoms,
wherein said glycerol mono-, di-, or triester is present in an amount imparting osteogenic properties for the composition.

2. A resorbable polymer composition of claim 1, wherein said glycerol mono-, di-, or triester is selected from glyceryl triacetate and glyceryl diacetate.

3. A resorbable polymer composition of claim 1, wherein said glycerol mono-, di-, or triester is glyceryl triacetate.

4. A resorbable polymer composition of claim 1, wherein said polymer matrix is selected from a group consisting of polyethylene glycols, polyglycolide, polylactides, polycaprolactones, polytrimethylenecarbonates, polyhydroxybutyrates, polyhydroxyvalerates, polydioxanones, polyorthoesters, polycarbonates, polytyrosinecarbonates, polyorthocarbonates, polyalkylene oxalates, polyalkylene succinates, poly(malic acid), poly(maleic anhydride), polypeptides, polydepsipeptides, polyvinylalcohol, polyesteramides, polyamides, polyanhydrides, polyurethanes, polyphosphazenes, polycyanoacrylates, polyfumarates, poly(amino acids), modified polysaccharides, modified proteins and their copolymers, terpolymers or combinations or mixtures or polymer blends thereof.

5. A resorbable polymer composition of claim 1, wherein the polymer matrix is selected from the group consisting of polyglycolide, poly(L-lactide-co-glycolide), poly(D,L-lactide-co-glycolide), poly(L-lactide), poly(D,L-lactide), poly(L-lactide-co-D,L-lactide), polycaprolactone, poly(L-lactide-co-caprolactone), poly(D,L-lactide-co-caprolactone) polytrimethylenecarbonate, poly(L-lactide-co-trimethylenecarbonate), poly(D,L-lactide-co-trimethylenecarbonate), polydioxanone and their copolymers, terpolymers or combinations or mixtures or polymer blends thereof.

6. The resorbable polymer composition of claim 1, wherein said glycerol mono-, di-, or triester is present in an amount between 0.05 and 60 weight-%.

7. A resorbable implant having osteogenic properties, comprising:
a base material including polymer matrix of resorbable polymer or copolymer, and a glycerol mono-, di-, or triester with a carboxylic acid having 1 to 6 carbon atoms.

8. A resorbable implant of claim 7, wherein said glycerol mono-, di-, or triester is selected from glyceryl triacetate and glyceryl diacetate.

9. A resorbable implant of claim 7, wherein said glycerol mono-, di-, or triester is glyceryl triacetate.

10. The resorbable implant of claim 7, wherein the polymer matrix is selected from a group consisting of polyglycolide, polylactides, polycaprolactones, polytrimethylenecarbonates, polyhydroxybutyrates, polyhydroxyvalerates, polydioxanones, polyorthoesters, polycarbonates, polytyrosinecarbonates, polyorthocarbonates, polyalkylene oxalates, polyalkylene succinates, poly(malic acid), poly(maleic anhydride), polypeptides, polydepsipeptides, polyvinylalcohol, polyesteramides, polyamides, polyanhydrides, polyurethanes, polyphosphazenes, polycyanoacrylates, polyfumarates, poly(amino acids), modified polysaccharides, modified proteins and their copolymers, terpolymers or combinations or mixtures or polymer blends thereof.

11. The resorbable implant of claim 7, wherein the polymer matrix is selected from a group consisting of polyglycolide, poly(L-lactide-co-glycolide), poly(D,L-lactide-co-glycolide), poly(L-lactide), poly(D,L-lactide), poly(L-lactide-coD,L-lactide), polycaprolactone, poly(L-lactide-co-caprolactone), poly(D,L-lactide-co-caprolactone) polytrimethylenecarbonate, poly(L-lactide-co-trimethylenecarbonate), poly(D,L-lactide-co-trimethylenecarbonate), polydioxanone and their copolymers, terpolymers or combinations or mixtures or polymer blends thereof.

12. The resorbable implant having osteogenic properties of claim 7, wherein said glycerol mono-, di-, or triester is present in an amount between 0.05 and 50 weight-%.

13. A method of making an implant having osteogenic properties comprising the steps of:
selecting polymer(s) or copolymer(s) of a polymer matrix of the implant,
adding a glycerol mono-, di-, or triester with a carboxylic acid having 1 to 6 carbon atoms to the polymer matrix in an amount imparting osteogenic properties for the implant,
forming the implant from the mixture of said polymer matrix and said glycerol mono-, di-, or triester.

14. A method of claim 13, wherein said glycerol mono-, di-, or triester is glyceryl triacetate.

15. A method of making an implant having osteogenic properties comprising the steps of:
selecting polymer(s) or copolymer(s) of a polymer matrix of the implant,
mixing said polymer(s) or copolymer(s) to form the polymer matrix,
forming the implant from said polymer matrix,
adding a glycerol mono-, di-, or triester with a carboxylic acid having 1 to 6 carbon atoms to the implant in an amount imparting osteogenic properties for said implant.

16. A method of claim 15, wherein said glycerol mono-, di-, or triester is selected from glyceryl triacetate and glyceryl diacetate.

17. A method of claim 15, wherein said glycerol mono-, di-, or triester is glyceryl triacetate.

18. The method of claim 15, wherein said glycerol mono-, di-, or triester is triacetin and wherein glyceryl triacetate is added to the implant preoperatively.

19. The method of claim 15, wherein the glycerol mono-, di-, or triester derivatives are chemically coupled to the implant.
